(19) Europäisches Patentamt
European Patent Office
Office européen des brevets

(11) Publication number: **0 207 714 B1**

# (12) EUROPEAN PATENT SPECIFICATION

(45) Date of publication of patent specification: **04.09.91** (51) Int. Cl.5: **C08F 220/06**

(21) Application number: **86304855.9**

(22) Date of filing: **24.06.86**

## (54) Improved process for preparing water-absorbing resins.

(30) Priority: **24.06.85 US 748246**
**25.06.85 US 748528**

(43) Date of publication of application:
**07.01.87 Bulletin 87/02**

(45) Publication of the grant of the patent:
**04.09.91 Bulletin 91/36**

(84) Designated Contracting States:
**AT BE CH DE FR GB IT LI NL SE**

(56) References cited:
**GB-A- 1 337 084**

**CHEMICAL ABSTRACTS, vol. 88, 03 August 1978, Columbus, OH (US); p. 29, no. 90451s**

(73) Proprietor: **AMERICAN COLLOID COMPANY**
**5100 Suffield Court**
**Skokie Illinois 60077(US)**

(72) Inventor: **Alexander, William**
**242 Terrance**
**Naperville, IL 60540(US)**
Inventor: **Teppo, Maynard**
**244 Belle Fourche**
**5717, SD(US)**
Inventor: **Anderson, Mark**
**500 N. Carlton**
**Wheaton, IL 60187(US)**

(74) Representative: **Wain, Christopher Paul et al**
**A.A. THORNTON & CO. Northumberland House 303-306 High Holborn**
**London WC1V 7LE(GB)**

**EP 0 207 714 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a method of manufacturing polyacrylate resins having improved water absorbing properties and more particularly to an improved process of preparing cross-linked polymers of acrylic acid and polyvinyl monomers.

Water absorbing resins have found wide use in sanitary goods, hygenic goods, water retaining agents, dehydrating agents, sludge coagulants, thickening agents, condensation preventing agents and release control agents for various chemicals. Water absorbing resins heretofore known include hydrolysis products of starch-acrylonitrile graft polymers, carboxymethylcellulose, cross-linked polyacrylate products and other resins such as polyvinyl alcohol, polyethylene oxide and polyacrylonitrile resins. Of these water absorbing resins, the hydrolysis products of starch and acrylonitrile graft polymers have comparatively high ability to absorb water but require a cumbersome process for production and have the drawbacks of low heat resistance and decaying or decomposing easily due to the presence of starch.

One of the processes for polymerizing acrylic acid and acrylates is aqueous solution polymerization. The polymer obtained by this process is soluble in water and, therefore, is cross-linked to modify the polymer into a useful water absorbing resin. However, even if the modification is effected by reacting a cross-linking agent concurrently with or after aqueous solution polymerization, the resulting reaction product is in the form of a highly viscous aqueous solution or a gel containing absorbed water which is difficult to handle. Thus, the aqueous solution or gel must be dehydrated (dried) to obtain a water absorbing resin in the desired solid or powder form. It is nevertheless difficult to dry the reaction product efficiently by the usual rotary drum roller method or spray drying method because care must be taken to avoid excessive cross-linking which results from over-heating during drying and insufficient drying results in reduced cross-linking density. Extreme difficulties are therefore encountered in preparing a product of a desired low water content and good water absorbing ability.

An object of the present invention is to provide a process for preparing a water absorbing cross-linked acrylate resin of low water content by aqueous solution polymerization without any additional dehydrating or drying step.

Another object of the present invention is to provide a process for preparing a cross-linked polyacrylate resin by polymerization of acrylic acid neutralized 70-100 mole percent, and a water-miscible to water soluble polyvinyl monomer in a combined concentration of 30 to 80 wt. % in water and initiating polymerization without external heating.

Another object of the present invention is to provide a process for preparing a cross-linked polyacrylate resin by co-polymerization of acrylic acid neutralized 70-100 mole percent, with acrylamide and a polyvinyl monomer in proportions of 0 to 30 mole percent acrylamide and 70-100 mole percent neutralized acrylic acid.

Another object of the present invention is to provide a process for producing a polyacrylate resin cross-linked with .2 weight percent to .6 weight percent based on the weight of monomers, of a water miscible or water soluble polyvinyl monomer cross-linking agent to achieve a "dry feel" to the resin after significant water absorption.

In brief, the present invention is directed to a process for preparing water absorbing, cross-linked acrylate resins by aqueous polymerization of (A) acrylic acid neutralized 70 to 100 mole percent for example with ammonia, and/or caustic alkali and/or an amine; with (B) acrylamide in a mole ratio of 70 to 100 mole percent (A) to 30:0 mole percent (B); and (C) a water miscible or a water soluble polyvinyl monomer in an amount of .001 to 0.3 weight percent based on the total weight of (A) and (B). To achieve the full advantage of the present invention the monomer concentration is at least 50 wt. % of the aqueous solution. A "dry feel" is obtained at a polyvinyl monomer concentration of at least .2 wt. % of the aqueous solution.

In accordance with the present invention, there is provided a process for preparing a solid water absorbing resin characterised in that it comprises mixing a monomer solution of (A) acrylic acid, 70-100 mole percent neutralized; and (B) a water-miscible to water-soluble polyvinyl monomer; in a combined concentration of at least 30 wt. % with water to form a mixed monomer solution, and initiating polymerization of monomers (A) and (B) such that during polymerization, the exothermic heat of reaction is substantially the only heat energy used to accomplish polymerization, cross-linking and to drive off sufficient water to obtain a solid cross-linked resin having a water content of 15 percent by weight or less.

It has been found that acrylic acid neutralized in the range of 70 to 100 mole percent will polymerize and cross-link rapidly with a polyvinyl monomer cross-linking agent to drive away excess water leaving a solid water absorbing resin having a desired degree of polymerization as well as new and unexpected water absorbing capacity. One or more polymerization catalysts or initiators can be added to the aqueous

monomer mixture to aid in polymerization.

A hot aqueous solution is prepared first which comprises acrylic acid neutralized 70 to 100 mole percent, a water-miscible or water-soluble polyvinyl monomer, water and, when desired, an organic solvent having a boiling point of 40 to 150° C, and which contains the acrylate monomer and the polyvinyl monomer in a combined concentration of 30 to 80 wt. %. To achieve the full advantage of the present invention, the acrylate and polyvinyl monomers are present in a combined concentration of less than 70 in weight percent of the monomer solution. In accordance with another important embodiment of the present invention, the combined concentration of the acrylate and polyvinyl monomers is less than 55 weight percent of the monomer solution. The concentration of the monomers is deliberately determined considering the state of the solution (i.e. as to whether or not the monomers can be completely dissolved in water), ease of the reaction of the monomers, escape of the monomers due to the scattering during the reaction, etc. The aqueous solution can be prepared easily usually by placing acrylic acid, a strong alkali such as potassium hydroxide and/or ammonium hydroxide or a basic amine for neutralizing the acid and the polyvinyl monomer into water in such amounts that the resulting solution has the above-mentioned monomer concentration. To dissolve the monomers thoroughly, the mixture can be heated to an elevated temperature. Any strongly basic alkali metal compound can be used for neutralization of the acrylic acid, such as potassium hydroxide, sodium hydroxide, lithium hydroxide, cesium hydroxide, potassium carbonate or sodium carbonate. Although it is desirable to use the neutralizing agent usually in an amount sufficient to neutralize acrylic acid 100 mole %, there is no particular need to neutralize the acid 100% insofar as the neutralizing agent, e.g., hydroxide, is used in such an amount as to achieve not less than about 70% neutralization. Accordingly the aqueous solution may contain up to about 30% of free acrylic acid. However, a large quantity of free acrylic acid, if present in the aqueous solution, is likely to partly splash out of the system to result in a loss during the reaction, leading to a reduced degree of polymerisation. Use of an excessive amount of the neutralizing agent will not raise any particular problem, but the excess does not participate in the polymerization reaction and is therefore useless.

We have also found that when the aqueous solution further contains an organic solvent having a boiling point of 40 to 150° C, the temperature of the aqueous solution is controllable with great ease and the resulting cross-linked resin has remarkably improved ability to absorb water at an initial rate.

When incorporating an organic solvent according to the invention, the aqueous monomer solution has a solidifying point which is about 10 to about 20° C lower than otherwise. This increases the allowable range of temperature control at least about 3 times. The organic solvent used is vigorously evaporated along with water by the heat of polymerization of the monomer. Since the latent heat of the evaporation is considerably smaller than that of water, the organic solvent functions as a blowing agent in the polymerization reaction system, consequently rendering the resulting resin porous. The resin exhibits about 2 to about 5 times higher initial rate of water absorption than the one obtained without using the organic solvent while possessing high water absorbing ability.

Thus, the organic solvent, when added to the aqueous monomer solution, produces improved effects without in any way impairing the advantages resulting from the use of the monomer solution.

Examples of organic solvents to be used in the invention when desired and having a boiling point of 40 to 150° C are methanol, ethanol, propanol and like alcohol solvents, acetone, methyl ethyl ketone and like ketone solvents, cyclohexane, n-hexane, n-heptane and like hydrocarbon solvents, benzene, toluene and like aromatic hydrocarbon solvents, and tetrahydrofuran and like furan solvents. These solvents may be used singly or in admixture. The solvent is used in an amount of 0.5 to 15 wt. %, preferably 1 to 10 wt. %, based on the combined amount of the monomers. With less than 0.5 wt. % of the solvent present, a sufficient blowing action will not take place, while the solidifying point of the monomer solution will not lower greatly. Conversely if more than 15 wt. % of the solvent isused, the resulting resin is likely to exhibit reduced water absorbing ability although achieving a high initial rate of water absorption. Moreover the monomers are likely to separate out, hence objectionable. Because the monomer solutiuon is heated prior to polymerization and further because the organic solvent evaporates along with water, the boiling point of the solvent is more preferably in the range of 55 to 120° c.

In accordance with the present invention, acrylic acid neutralized 70-100 mole percent is mixed with a water-miscible or water-soluble polyvinyl monomer in an aqueous solution at a temperature of about 20 to 100° C. The solution is subjected to a polymerization reaction and a cross-linking reaction by the addition of a polymerization initiator. The polymerization reaction proceeds sufficiently within a very short period of time and if the monomer concentration is at least 30 percent by weight of the aqueous monomer mixture, the heat of the polymerization and cross-linking reactions will evaporate water rapidly from the reaction system to form a dry solid (less than 15 percent by weight water) water absorbing resin without the need for any subsequent drying step. The solid can be easily pulverized into a powder suitable for any desired use.

According to the process of the invention, a hot, i.e. at least 25° C., aqueous solution is prepared first including acrylic acid neutralized 70 to 100 mole percent, optionally acrylamide, a water-miscible or water-soluble polyvinyl monomer, and water. The aqueous solution can be prepared easily by placing (A) acrylic acid, and an amine, and/or a caustic alkali and/or ammonia for neutralizing the acid; (B) acrylamide (0-30 mole percent); and (C) a polyvinyl monomer into water to form a mixed monomer solution. To dissolve the monomers thoroughly, the mixture can be heated to an elevated temperature up to the boiling point of water i.e. 100° C.

The polyvinyl monomer to be used in both embodiments of the invention should be miscible with or soluble in water so that the monomer will be uniformly dissolved or dispersed in the aqueous solution of the monomer mixture. Examples of such polyvinyl monomers include bisacrylamides such as N,N'-methylenebisacrylamide and N,N'-methylenebismethacrylamide; polyacrylic (or polymethacrylic) acid esters represented by the following formula (I); and diacrylamides represented by the following formula (II). Among these, especially preferably are N,N'-methylenebisacrylamide, N,N'-methylenebismethacrylamide and like bisacrylamides. Formula (I)

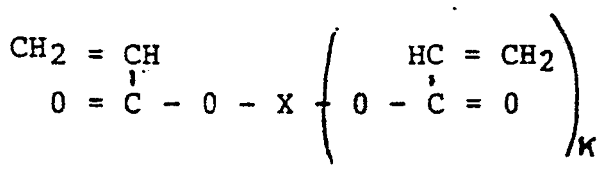

wherein X is ethylene, propylene, trimethylene, hexamethylene, 2-hydroxypropylene,$(CH_2CH_2O)_nCH_2CH_2$ - or

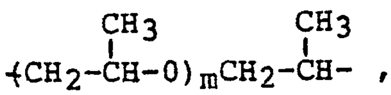

n and m are each an integer of from 5 to 40, and k is 1 or 2.

The compounds of the formula (I) are prepared by reacting polyols, such as ethylene glycol, propylene glycol, trimethylolpropane, 1,6-hexanediol, glycerin, pentaerythritol, polyethylene glycol and polypropylene glycol, with acrylic acid or methacrylic acid. Formula (II):

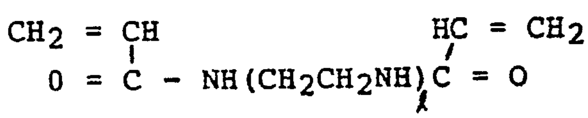

wherein ℓ is 2 or 3.

The compounds of the formula (II) are obtained by reacting polyalkylenepolyamines, such as diethylenetriamine and triethylenetetramine, with acrylic acid.

The polyvinyl monomer is used in an amount of about 0.001 to 0.3 wt. % of the amount of acrylic monomers in the aqueous monomer mixture. In accordance with an important embodiment of the present invention, the polyvinyl monomer should be present in the aqueous solution in an amount of at least .2 wt. % based on the total weight of monomers to provide a resin sufficiently cross-linked to have a "dry feel" after significant water absorption. If the polyvinyl monomer is included in the aqueous solution in an amount of .2 to .6 weight percent based on the weight of neutralized acrylic acid and polyvinyl monomers, the resulting polymer will have an exceedingly "dry feel" cn absorption of water.

The aqueous mixed monomer solution is heated and thereafter subjected to polymerization and cross-linking reactions with the addition of a polymerization initiator. Although the temperature of the aqueous mixed monomer solution is not particularly limited since the mixed monomer solution is initiated into polymerization by the addition of the initiator, the temperature is usually about 50 to about 85° C, preferably about 60 to about 75° C. Various polymerization initiators are usable which are known for use in preparing polyacrylates. Examples of useful initiators are redox initiators comprising a reducing agent, such

as a sulfite or bisulfite of an alkali metal, ammonium sulfite or ammonium bisulfite, and an initiator, such as a persulfate of an alkali metal or ammonium persulfate, in combination with the reducing agent; azo initiators including azobis-isobutyronitrile, 4-t-butylazo-4'-cyanovaleric acid, 4,4'-azobis(4-cyanovaleric acid) and 2,2'-azobis(2-amidinopropane)-hydrochloric acid salt; and trimethylolpropane triacrylate.

These initiators can be used singly or in a suitable combination. Of these, especially preferable are a redox initiator composed of ammonium persulfate and sodium hydrogensulfite, and azo initiators such a azobisisobutyronitrile and 2,2'-azobis(2-amidinopropane)-hydrochloric acid. These initiators are advantageously used usually in the form of an aqueous solution but can be used as diluted with a suitable solvent. The initiator is used in a usual amount, i.e. in an amount, calculated as solids, of about 0.1 to about 10%, preferably about 0.5 to about 5%, of the combined weight of the monomers, namely acrylate (and free acrylic acid), acrylamide, and polyvinyl monomer. Depending on the amount and kind of the initiator, the initiator is usable together with isopropyl alcohol, alkylmercaptan or other chain transfer agents to control the molecular weight of the polyacrylate to be obtained.

By the addition of the polymerization initiator, the mixed monomer solution is subjected to polymerization with evaporation of water without heating the system from outside. More advantageously, the reaction is carried out by admixing a predetermined amount of the initiator or an aqueous solution thereof with the mixed monomer solution and causing the resulting mixture to flow down onto and spread over a traveling conveyor belt. The initiator can be added to the mixed monomer solution as it is poured onto the conveyor belt.

The polymerization proceeds rapidly after admixing the initiator with the mixed monomer solution and is completed within a short period of time, usually in about 30 seconds to about 10 minutes. The reaction is exothermic, so that the reaction system is rapidly heated to about 100 to about 130° C by the heat of polymerization. Consequently, particularly where the monomer concentration in the mixed solution is at least 50 percent by weight, the water evaporates from the system rapidly to give a relatively dry, solid polymer of low water content without resorting to any external heating. The water content of the polymer is usually up to about 15%, and generally about 8 to 12% by weight e.g about 10% by weight as recovered. Subsequently, the dry solid polymer can be made into the desired powder easily by a usual method, for example by pulverization, without a drying step.

In accordance with another important feature of the present invention, polystyrene and/or methylcellulose can be added to the mixed monomer solution in an amount of 0.5 to about 10 percent based on the total weight of monomers in the mixed monomer solution to increase the porosity and water absorbing capacity of the polymers. It has been found, quite surprisingly, the polystyrene and methylcellulose will substantially increase the water absorbing capacity of the resin described herein. To achieve the full advantage of the present invention, the polystyrene and methylcellulose should be added in an average grain size of less than or equal to 5 micrometers.

The powder thus obtained has outstanding water absorbing ability and is useful for sanitary goods, paper diaper, disposable diaper and like hygenic goods, agricultural or horticultural water retaining agents, industrial dehydrating agents, sludge coagulants, thickening agents, condensation preventing agents for building materials, release control agents for chemicals and various other applications.

According to another aspect of the invention there is provided a method of absorbing water comprising mixing a monomer solution of (A) acrylic acid, 70 to 100 mole percent neutralized, (B) acrylamide in a mole ratio of acrylic acid: acrylamide in the range of 70:30 to 100:0; (C) a water soluble or water miscible polyvinyl monomer cross-linking agent in an amount of .001 to 0.3 percent by weight of (A) plus (B); and water to form a mixed monomer solution, in which the monomer concentration is below 70 percent by weight of the monomer solution prior to polymerization; and initiating polymerization such that during polymerization, the exothermic heat of reaction is substantially the only heat energy used to accomplish polymerization, cross-linking and to drive off sufficient water to form a water absorbing cross-linked polyacrylate resin having a water content of 15 percent by weight or less, and thereafter contacting said resin with water to absorb water into said resin.

The present invention will be described in greater detail with reference to the following examples.

Example 1

To deionized water are added, wherein percents are weight percents based on the total weight of the monomer solution formed, 58.81% acrylic acid first, then 11.76% potassium hydroxide and 11.76% ammonium carbonate and 14.70% ammonium hydroxide serving as neutralizing agents. Thereafter .03% of N,N-methylenebisacrylamide as a polyvinyl monomer is added to prepare an aqueous solution of potassium acrylate and ammonium acrylate in 2.79% of water having a neutralization degree of about 90% and a

combined monomer concentration of 58.84 wt.%.

The aqueous solution is maintained at 70°C, and with the solution are admixed 0.15% of 2,2-azobis-(2-amidino-propane)hydrochloric acid. The final solution is as follows:

| CHEMICALS | | |
|---|---|---|
| ACRYLIC ACID | | 58.81% |
| POTASSIUM HYDROXIDE | | 11.76% |
| AMMONIUM CARBONATE | | 11.76% |
| N,N-METHYLENEBISACRYLAMIDE | | 0.03% |
| POLYMERIZATION INITIATOR | | 0.15% |
| AMMONIUM HYDROXIDE | | 14.70% |
| $H_20$ | | 2.79% |
| | TOTAL | 100.00 |

The mixture is poured onto a traveling endless belt and spread thereover in the form of a layer about 10 mm in thickness. About 30 seconds thereafter, the mixture starts to polymerize, and the reaction is completed in about 1 minute. The maximum temperature of the mixture during the reaction is about 120° C.

The polymer is allowed to complete curing for about 30 minutes at ambient temperature to give a dry solid strip of cross-linked potassium polyacrylate product having a water content of 11% and a residual monomer concentration of 1200 ppm. The strip is made into a powder by a pulverizer.

Examples 2 to 4

Polymers are prepared in the same manner as in Example 1 with the exception of varying, at least one of the combined concentration of monomers, the amount of polyvinyl monomer (N,N-methylenebisacrylamide), the kind and amount (degree of neutralization) of neutralizing agent, and the amounts, based on the combined amount of the monomers, of azo polymerization initiator. The following compositions were polymerized:

| | EXAMPLE 2 | |
|---|---|---|
| ACRYLIC ACID | | 56.80% |
| POTASSIUM HYDROXIDE | | 14.77% |
| AMMONIUM CARBONATE | | 11.36% |
| N,N-METHYLENEBISACRYLAMIDE | | 0.03% |
| V-50 | | 0.14% |
| AMMONIUM HYDROXIDE | | 14.20% |
| H2O | | 2.70% |
| | TOTAL | 100.00% |

EXAMPLE 3

| | |
|---|---|
| ACRYLIC ACID | 57.13% |
| POTASSIUM HYDROXIDE | 14.28% |
| AMMONIUM CARBONATE | 11.43% |
| N,N-METHYLENEBISACRYLAMIDE | 0.03% |
| AZO POLYMERIZATION INITIATOR | 0.14% |
| AMMONIUM HYDROXIDE | 14.28% |
| H2O | 2.71% |
| TOTAL | 100.00% |

EXAMPLE 4

| | |
|---|---|
| ACRYLIC ACID | 54.66% |
| POTASSIUM HYDROXIDE | 10.93% |
| AMMONIUM CARBONATE | |
| N, N-METHYLENEBISACRYLAMIDE | 0.11% |
| V-50 | 0.41% |
| AMMONIUM HYDROXIDE | 30.61% |
| H2O | 3.27% |
| TOTAL | 100.00% |

The amount of polyvinyl monomer listed as expressed in % by weight based on the combined amount of potassium acrylate, free acrylic acid and the polyvinyl monomer, and the concentration of initiator is expressed in % by weight based on the combined amount by weight (calculated as solids) of the monomers and the initiator, the same as hereinbefore.

EXAMPLE 5

To 22.2 g of deionized water are added 72.1 g of acrylic acid first, then 49.5 g of potassium hydroxide having a purity of 85% and serving as a neutralizing agent, and thereafter 0.01 g of N,N-methylenebisacrylamide as a polyvinyl monomer to prepare an aqueous solution of potassium acrylate having a neutralization degree of 75% and a combined monomer concentration of 70 wt.%.

The aqueous solution is maintained at 70° C, and with the solution are admixed 2.9 g of 18% aqueous solution of ammonium persulfate (0.5 wt. % based on the combined weight of the potassium acrylate, free acrylic acid and N,N-methylenebisacrylamide, the same as hereinafter). The mixture is poured onto a traveling endless belt and spread thereover in the form of a layer about 10 mm in thickness. About 30 seconds thereafter, the mixture starts to polymerize, and the reaction is completed in about 1 minute. The maximum temperature of the mixture during the reaction is about 120° C.

The reaction gives a dry solid strip of cross-linked potassium polyacrylate product having a water content of 11% and a residual monomer concentration of 1200 ppm. The strip is made into a powder by a pulverizer. The powder has water absorbing ability of 450 as measured with use of deionized water or 60 as measured with 1% saline.

Examples 6 to 9

Polymers are prepared in the same manner as in Example 5 with the exception of changing at least one of the amount of N,N-methylenebisacrylamide and the kind and amount of the polymerization initiator as listed in Table 1 below. Table 1 also shows the water content and water absorbing ability of each polymer obtained.

Table 1

| Ex. No. | Initiator Kind | Initiator Conc. | Amt. of Polyvinyl Monomer | Water Content | Water Absorbing Ability Deionized Water | Water Absorbing Ability 1% Saline |
|---|---|---|---|---|---|---|
| | 2,2'-azobis-(2-amidino-propane) hydrochloric acid | | | | | |
| 6 | | 0.5 | 0.01 | 11 | 520 | 58 |
| 7 | " | 0.5 | 0.02 | 12 | 610 | 65 |
| 8 | " | 1.0 | 0.01 | 10 | 550 | 62 |
| 9 | " | 1.0 | 0.02 | 11 | 580 | 63 |

Examples 10 to 17

Polymers are prepared in the same manner as in Example 1 except that the compounds listed in Table 2 below are used as polyvinyl monomers in the listed amounts. Table 2 also shows the water content and water absorbing ability of each polymer obtained.

Table 2

| Ex. No. | Polyvinyl Monomer Kind | Amount | Water Content | Deionized Water Absorbing Ability |
|---|---|---|---|---|
| 10 | Ethylene glycol diallyl ester | 0.01 | 12 | 480 |
| 11 | " | 0.02 | 13 | 430 |
| 12 | Deithylenetri-amine-diacryl-amid | 0.01 | 12 | 510 |
| 13 | " | 0.02 | 12 | 450 |
| 14 | N,N-methylene-bismethacryl-amid | 0.01 | 9 | 520 |
| 15 | " | 0.05 | 11 | 390 |
| 16 | Polyethylene glycol diacrylate* | 0.01 | 10 | 500 |
| 17 | " | 0.05 | 11 | 430 |

*Polyethylene glycol diacrylate used in Examples 20 and 21 is represented by the following formula:

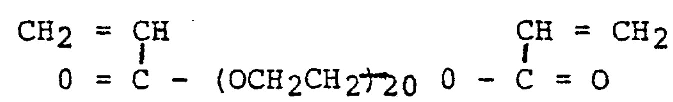

## Examples 18 to 21

Acrylic acid (72.1 g), 18.0 g of deionized water, 40.9 g of solid potassium hydroxide (water content 4%) and 5.2 g of one of the solvents (5 wt. % based on the monomers) listed in Table 3 are mixed together, and the mixture is maintained at 75° C. With the mixture is further admixed 4.0 g of 10% aqueous solution of 2,2'-azobis(2-amidinopropane)hydrochloric acid salt. The resulting mixture is immediately poured onto a traveling endless belt and spread thereover to a thickness of 5 mm. About 15 seconds later, the mixture starts to polymerize, and the polymerization is completed in about 30 seconds. The maximum temperature of the mixture during the reaction is 130 to 135° C.

The reaction gives a dry strip of cross-linked potassium polyacrylate product, which is pulverized to a powder 20 to 100 mesh in particle size.

The same procedure as above is repeated with use of the other solvents. All the powders obtained have a water content of 4 to 6%.

A 0.1 g quantity of each of the powders is accurately measured out and the water absorbing ability of the powder is measured after immersing the powder in deionized water or 1% saline for 10 seconds, 30 seconds or 15 minutes. Table 6 shows the results.

## Example 22

An aqueous monomer solution is prepared in the same manner as in examples 17 to 21 with the exception of not using any organic solvent and using 23.2 g of deionized water. The solution is thereafter subjected to polymerization in the same manner as in these examples to obtain a powder of dry solid. Table 3 also shows the test results obtained with this powder.

Table 3

| Example No. Organic Solvent | 18 Acetone | 19 Ethanol | 20 Benzene | 21 Tetra-hydro-furan | 22 (Water only) |
|---|---|---|---|---|---|
| Water absorbing ability (times) | | | | | |
| 1% Saline | | | | | |
| 10 Sec. | 73 | 68 | 70 | 76 | 54 |
| 30 Sec. | 83 | 82 | 85 | 85 | 75 |
| 15 Min. | 97 | 93 | 94 | 96 | 96 |
| Deionized water | | | | | |
| 10 Sec. | 620 | 690 | 600 | 690 | 300 |
| 30 Sec. | 900 | 910 | 880 | 920 | 750 |
| 15 Min. | 960 | 980 | 900 | 980 | 920 |

Examples 23 to 25

Water absorbing resin solids are prepared in the same manner as in Example 18 with the exception of using 3, 5 or 10 wt. %, based on the monomers, of methanol in place of 5.2 g of acetone and varying the amount of deionized water so that the combined amount of the water and the methanol is 23.2 g. Each of the solids is tested for water content and also for water absorbing ability by immersion in 1% saline and deionized water for specified periods of time. The results are given in Table 4, which also shows the resluts of similar tests conducted with use of the solid of Example 22.

Table 4

| Example No. | 19 | 20 | 21 | 22 |
|---|---|---|---|---|
| Amount of organic solvent (based on monomers, %) | 3 | 5 | 10 | 0 |
| Water content of resin | 7.2 | 6.0 | 2.1 | 10.2 |
| Water absorbing ability (times) | | | | |
| 1% Saline | | | | |
| 5 Sec. | 50 | 58 | 65 | 32 |
| 10 Sec. | 64 | 71 | 75 | 54 |
| 15 Min. | 71 | 77 | 79 | 61 |
| 20 Sec. | 75 | 80 | 82 | 67 |
| 40 Sec. | 85 | 87 | 88 | 80 |
| 60 Sec. | 90 | 90 | 91 | 88 |
| 15 Min. | 97 | 93 | 94 | 96 |
| Deionized water | | | | |
| 5 Sec. | 250 | 420 | 570 | 140 |
| 10 Sec. | 420 | 650 | 760 | 300 |
| 15 Sec. | 590 | 740 | 830 | 410 |
| 20 Sec. | 660 | 790 | 850 | 560 |
| 40 Sec. | 880 | 900 | 890 | 850 |
| 60 Sec. | 920 | 910 | 890 | 900 |
| 15 Min. | 930 | 910 | 890 | 920 |

Example 26

281. gr. of acrylic acid and 11.9 gr. of acrylamide were dissolved in 179.1 gr. of distilled water and then 10.9 gr. of NaOH was added for 70 mole percent partial neutralization of acrylic acid; 0.003 gr. of N,N'-methylenebisacrylamide was then added as the polyvinyl monomer. In this case, 0.04 gr. of 2,2,-azobis(2-amidinopropane)hydrochloride was added as the polymerization initiator and the initial temperature of the mixed monomer solution was 50° C.

Example 27

48.1 gr. of acrylic acid and 11.9 gr. of acrylamide were dissolved in 159 gr. of distilled water and then 22.7 gr.of NaOH was added for partial neutralization of acrylic acid in an amount of 85 mole percent. 0.006 gr. of N,N'-methylenebisacrylamide was then added as the polyvinyl monomer. In addition, 0.048 gr. of ammonium persulfate and 0.048 gr. of sodium hydrogen-sulfite were added as the polymerization initiators. In this case, the initial temperature of the mixed monomer solution was 40° C.

Example 28

90.1 gr.of acrylic acid and 9.9 gr. of acrylamide were dissolved in 118.8 gr. of distilled water and 52.6 gr. of KOH was added for 75 mole percent partial neutralization of acrylic acid. 0.018 gr. of N,N'-methylenebisacrylamide was added as the polyvinyl monomer, and 0.08 gr. of ammonium persulfate and 0.08 gr. of sodium hydrogen-sulfite were added as the polymerization initiators. The initial temperature of the mixed monomer solution was 30° C.

Example 29

48.1 gr. of acrylic acid and 11.9 gr. of acrylamide were dissolved in 252.1 gr. of distilled water and 27.4 gr. of aqueous ammonia was added for 70 mole percent partial neutralization of acrylic acid. In this case, the concentration of the ammonia is 29 weight percent. In addition, 0.006 gr. of N,N'-methylenebisacrylamide was added as the polyvinyl monomer. The polymerization was performed with the addition of 0.048 gr. of ammonium persulfate and 0.048 gr. of sodium hydrogen-sulfite for initiation. In this case, the initial temperature of the mixed monomer solution was 30° C.

Example 30

52.7 gr. of acrylic acid and 17.3 gr. of acrylamide were dissolved in 25 gr. of distilled water and they were partially neutralized 80 mole percent with the addition of 32.8 gr. of KOH. 0.007 gr. of N,N'-methylenebisacrylamide was added as to the polyvinyl monomer. For the polymerization catalyst 0.7 gr. of 2,2'-azobisisobutyronitrile dissolved in 10 cc. of acetone was added. This solution was kept at 80° C. in a TEFLON coated, glass fiber reaction chamber until completion of polymerization and cross-linking reactions yielding a white, solid resin.

Example 31

17.3 gr of acrylamide was dissolved in 52.7 gr. of acrylic acid and partial (70 mole percent) neutralization of acrylic acid was accomplished by the addition of 30 gr. of aqueous ammonia having a concentration of 29 weight percent. In this case, for the polyvinyl monomer, 0.007 gr. of N,N'-methylenebisacrylamide was added and, as the catalyst, 0.7 gr. of 2,2'-azobis(2-amidinopropane)-hydrochloride dissolved in 8 gr. of distilled water was added. The polymerization was started at 80° C. yielding a white, solid resin.

Example 32

21 kg. of acrylic acid and 7 kg. of acrylamide were dissolved in 9.5 kg. of distilled water and the acrylic acid was partially neutralized with 12 kg. of KOH. 0.036 kg. of methylenebisacrylamide as a polyvinyl monomer was added to provide an aqueous mixed monomer solution. The mixed monomer solution was mixed with 0.28 kg. of 2,2'-azobisisobutyronitrile, dissolved in 2 kg. of acetone as a polymerization initiator. This mixture at a temperature of 60° C. was transferred on an endless belt (600-700 mm. in width, and 7 m. in length) at a thickness of about 1 cm. The polymerization was initiated promptly on the belt resulting in a white, solid resin.

Example 33

The polymers of Examples 26-29, were dehydrated with acetone, dried and pulverized into powders. The polymers of Examples 30-32, were pulverized without any drying into highly water absorbable powders. The products of Examples 26-32, were compared to commercial prior art products. The testing method was as follows:

1 gr. of sample was added to 1 liter of distilled water with agitation. After one hour of settling for water absorption, the water-polymer was filtered through a 100 mesh sieve and the amount of water absorbed determined by measuring the volume of filtrate recovered. In addition, parallelly, 5 gr. of the polymers of Examples 26-32, were added to 1 liter of 1 percent NaCl solution, and testing was conducted the same as in the case of distilled water. The results of the testing are shown in Table 5. Water absorbability is shown as amount of absorbed water/weight of resin.

Table 5

Examples 26-32

| | Distilled Water | 1% NaCl Solution |
|---|---|---|
| Example 26 | 650 times | 68 times |
| Example 27 | 670 times | 70 times |
| Example 28 | 680 times | 72 times |
| Example 29 | 620 times | 65 times |
| Example 30 | 540 times | 57 times |
| Example 31 | 520 times | 54 times |
| Example 32 | 540 times | 56 times |
| Commercial product (1) | 520 times | 49 times |
| Commercial product (2) | 504 times | 47 times |

Example 33

52.7 gr. of acrylic acid and 17.3 gr. of acrylamide were dissolved in 20 gr. of distilled water. 32.8 gr. of KOH was added for 80 mole percent partial neutralization of acrylic acid. Then 0.007 gr. of N,N'-methylenebisacrylamide polyvinyl monomer was added with 2.5 gr. of methylcellulose. The aqueous monomer mixture was homogenized with agitation. Next, as an initiator, 0.7 gr. of 2,2'-azobisisobutyronitrile dissolved in 10 cc of acetone was added. This mixed solution was kept at 80° C. in a water bath surrounding the reaction chamber. Polymerization was initiated as the temperature of the mixture increased as a result of the surrounding water bath, resulting in a white, solid porous resin.

Example 34

20.8 gr. of acrylamide was dissolved in 49.2 gr. of acrylic acid and 28.0 gr. of aqueous ammonia (29% concentration) was added for 70 mole percent neutralization of the acrylic acid. Next, 0.01 gr. of N,N'-methylenebisacrylamide and 5 gr. of methylcellulose were added and stirred to homogenize. As an initiator, 0.7 gr. of 2,2'-azobis(2-amidinopropane)hydrochloride dissolved in 5 gr. of distilled water, was added. The polymerization was initiated in a TEFLON coated glass reaction chamber kept at 80° C., as in Example 8 yielding a white, solid porous resin having a water content of about 10% by weight.

Example 35

70 gr. of acrylic acid was dissolved in 20 gr. of distilled water. 40.8 gr. of KOH was added to neutralize 75 mole percent of the acrylic acid. Next, 0.007 gr. of N,N'-methylenebisacrylamide and 2.0 gr. of methylcellulose were added, and the mixture homogenized with agitation. Then, as an initiator, 0.7 gr. of 2,2'-azobisisobutyronitrile dissolved in 10 cc. of acetone was added. This solution was transferred to the reaction chamber kept at 80° C. and polymerization was initaited with a rise in temperature of the mixture resulting in a white, solid porous resin of low water content.

Example 36

Same as Example No. 33, but having no methylcellulose.

Example 37

Same as Example No. 34, but having no methylcellulose.

Example 38

Same as Example No. 35, but having no methylcellulose.

Example 39

The water absorbable resins of Example 33-38 were pulverized directly into powder without any drying process.

The water absorbing capacity of the resins of Examples 33-38 was tested as follows:

1 gr. of the resin powders (20-40 mesh) of Examples 33-38 were each added to 2 liters of distileld water with agitation. After settling for 1 hour, the water-polymers were filtered through a 100 mesh sieve and the volume of filtrate measured to give the amount of water absorbed in the resin. In adition, 5 gr. of the resins of Examples 33-38 were each added to 1 liter of 1% NaCl solution, and the testing was conducted the same as the case of distilled water. The results are shown in Table 6. Water absorbability is shown as amount of absorbed water/weight of resin

Table 6
Examples 33-38

| | Distilled Water | 1% NaCl Solution |
|---|---|---|
| Example 33 | 1,300 times | 98 times |
| Example 34 | 1,160 times | 84 times |
| Example 35 | 1,360 times | 102 times |
| Example 36 | 540 times | 56 times |
| Example 37 | 470 times | 50 times |
| Example 38 | 590 times | 60 times |

Example 39

52.7 gr. of acrylic acid and 17.3 gr.of acrylamide were dissolved in 20 gr. of distilled water. 32.8 gr. of potassium hydroxide was added to neutralize 80 mole percent of the acrylic acid. Next, 0.007 gr. of N,N'-methylenebisacrylamide and 5 gr. of a polystyrene emulsion were added and homogenized under stirring. The polystyrene emulsion had a concentration of 50 weight percent and a polystyrene grain size of about 0.5 micro meters. Then, as the initiator, 0.7 gr. of 2,2'-azobisisobutyronitrile dissolved in 10 cc. of acetone solution was added. The mixed monomer solution was kept at 80° C. in the reaction chamber immersed in a water bath. With the increasing temperature of the mixed monomer solution polymerization was initiated resulting in a white, solid, porous resin.

Example 40

20.8 gr. of acrylamide was dissolved in 49.2 gr. of acrylic acid. 28.0 gr. of aqueous ammonia (29% concentration) was added to neutralize 70 mole percent of the acrylic acid. Then 0.01 gr. of N,N'-methylenebisacrylamide was added. 3 gr. of the polystyrene emulsion of Example 39, but having a 1 micro meter average grain size, was added and the mixed solution was homogenized with agitation. Next, as the initiator, 0.7 gr. of 2,2'-azobis(2-amidinopropane)hydrochloride dissolved in 5 gr. of distilled water was

added. The solution was kept at 80° C. as in Example 39 and polymerization was initiated with increased temperature resulting in a white, solid, porous resin.

Example 41

70 gr. of acrylic acid was dissolved in 20 gr. of distilled water and 40.8 gr. of KOH was added to neutralize 75 mole % of the acrylic acid. Next, 0.007 gr. of N,N'-methylenebisacrylamide was added. 2 gr. of the polystyrene emulsion of Example 14 (but having an average grain size of 5 micro meters), was added and the mixture homogenized. Next, as the initiator, 0.7 gr. of 2,2'-azobisisobutyronitrile dissolved in 10 cc of acetone was added. The mixed solution was kept at 80° C. in the water bath to initiate the polymerization resulting in a white, solid, porous resin.

Example 42

Same as Example 39, but having no polystyrene.

Example 43

Same as Example 40, but having no polystyrene.

Example 44

Same as Example 41, but having no polystyrene.

The polymers of Examples 39-44 were made into powdery resins without any drying step.

Example 45

The following tests were performed on the resins of Examples 39-44 to determine water absorbing capacity:

1 gr. of the resins of Examples 39-44 (20-40 mesh) were each added into 2 liter of distilled water with agitation and settling for one hour. After settling, a 100 mesh sieve was employed for filtration and the amount of absorbed water was calculated from the amount of filtrate. Their water absorption rate (absorbed water/weight of polymer) is shown in the following Table 7.

Table 7
Examples 39-44

| | Distilled Water | 1% NaCl Solution |
|---|---|---|
| Example 39 | 1,010 times | 83 times |
| Example 40 | 960 times | 76 times |
| Example 41 | 1,120 times | 90 times |
| Example 42 | 540 times | 56 times |
| Example 43 | 470 times | 50 times |
| Example 44 | 590 times | 60 times |

## Claims

1. A process for preparing a solid water absorbing resin characterised in that it comprises mixing a monomer solution of (A) acrylic acid, 70-100 mole percent neutralized; and (B) a water-miscible to water-soluble polyvinyl monomer; in a combined concentration of at least 30 wt. % with water to form a mixed monomer solution, and initiating polymerization of monomers (A) and (B) such that during polymerization, the exothermic heat of reaction is substantially the only heat energy used to accomplish

polymerization, cross-linking and to drive off sufficient water to obtain a solid cross-linked resin having a water content of 15 percent by weight or less.

2. A process as claimed in claim 1, characterised in that component (A) is potassium acrylate.

3. A process as claimed in either claim 1 or claim 2, characterised in that the combined concentration of the monomers (A) and (B) is at least 30 wt. % and less than 70 wt. %.

4. A process as claimed in any one of the preceding claims, characterised in that the mixed monomer solution has a temperature of 50 to 85° C prior to polymerization.

5. A process as claimed in any one of the preceding claims, characterised in that monomer (B) comprises N,N-methylenebisacrylamide or N,N-methylenebismethacrylamide.

6. A process as claimed in any one of the preceding claims, characterised in that the mixed monomer solution contains 1 to 10 wt. % of an organic solvent based on the weight of monomers (A) and (B).

7. A process as claimed in any one of the preceding claims, characterised in that the mixed monomer solution further contains an organic solvent having a boiling point of 40° to 150° C.

8. A process as claimed in any one of the preceding claims, characterised in that said polymerization initiator is added in an amount of at least 0.5% total by weight of monomers (A) and (B).

9. A process as claimed in any one of the preceding claims, characterised in that the water content of said cross-linked resin is not greater than about 10% by weight as recovered from the polymerized mixture, without an additional drying step.

10. A process as claimed in any one of the preceding claims, further characterised by the step of pulverizing said cross-linked resin to form a powder.

11. A process as claimed in any one of the preceding claims characterised in that the mixed monomer solution comprises (A) acrylic acid, 70 to 100 mole percent neutralized, (B) acrylamide in a mole ratio of (A):(B) in the range of 70:30 to 100:0; (C) a water soluble or water miscible polyvinyl monomer cross-linking agent in an amount of 0.001 to 0.3 percent by weight of (A) plus (B); and water, in which the monomer concentration is below 70 percent by weight of the monomer solution prior to polymerization initiation.

12. A process as claimed in claim 11, characterised in that the water content of said cross-linked polyacrylate resin is not greater than about 10% by weight as recovered from the mixed monomer solution after polymerization, without an additional drying step.

13. A process as claimed in any one of the preceding claims, characterised by depositing said monomer mixture including said initiator onto a support surface in sheet form for polymerization and cross-linking.

14. A method of absorbing water by preparing a solid water absorbing resin in accordance with any one of the preceding claims and thereafter contacting said resin with water to absorb water into said resin.

**Revendications**

1. Un procédé de préparation d'une résine solide absorbant l'eau, caractérisé en ce qu'il consiste à mélanger une solution monomère de (A) acide acrylique, neutralisé à 70-100 % molaire ; et (B) un monomère polyvinylique miscible à l'eau à solubte dans l'eau ; à une concentration combinée d'au moins 30 % en poids avec de l'eau pour former une solution monomère mixte, et à amorcer la polymérisation des monomères (A) et (B) de façon que durant la polymérisation la chaleur de la réaction exothermique soit substantiellement la seule énergie thermique utilisée pour accomplir la polymérisation, la réticulation et pour éliminer suffisamment d'eau pour obtenir une résine solide réticulée ayant une teneur en eau de 15 % en poids ou moins.

2. Un procédé selon la revendication 1, caractérisé en ce que le composant (A) est l'acrylate de potassium.

3. Un procédé selon la revendication 1 ou 2, caractérisé en ce que la concentration combinée des monomères (A) et (B) est d'au moins 30 % en poids et inférieure à 70 % en poids.

4. Un procédé selon l'une quelconque des revendications précédentes, caractérisé en ce que la solution monomère mixte a une température de 50 à 85° C avant la polymérisation.

5. Un procédé seton l'une quelconque des revendications précédentes, caractérisé en ce que le monomère (B) comprend le N,N-méthylènebisacrylamide ou le N,N-méthylènebisméthacrylamide.

6. Un procédé selon l'une quelconque des revendications précédentes, caractérisé en ce que la solution monomère mixte contient 1 à 10 % en poids d'un solvant organique basé sur le poids des monomères (A) et (B).

7. Un procédé selon l'une quelconque des revendications précédentes, caractérisé en ce que la solution monomère mixte contient en outre un solvant organique ayant un point d'ébullition de 40° à 150°C.

8. Un procédé selon l'une quelconque des revendications précédentes, caractérisé en ce que ledit amorceur de polymérisation est ajouté en quantité d'au moins 0,5 % du poids total des monomères (A) et (B).

9. Un procédé selon l'une quelconque des revendications précédentes, caractérisé en ce que la teneur en eau de ladite résine réticulée n'est pas supérieure à environ 10 % en poids à l'état récupéré du mélange polymérisé, sans étape additionnelle de séchage.

10. Un procédé selon l'une quelconque des revendications précédentes, caractérisé en outre par l'étape de pulvérisation de ladite résine réticulée pour former une poudre.

11. Un procédé selon l'une quelconque des revendications précédentes, caractérisé en ce que la solution monomère mixte comprend (A) l'acide acrylique, neutralisé à 70 à 100 % molaire, (B) l'acrylamide dans un rapport molaire de (A) : (B) dans l'intervalle de 70 : 30 à 100 : 0 ; (C) un agent de réticulation de monomère polyvinylique soluble dans l'eau ou miscible à l'eau en quantité de 0,001 à 0,3 % en poids de (A) plus (B) ; et de l'eau, dans laquelle la concentration du monomère est inférieure à 70 % en poids de la solution monomère avant l'amorçage de la polymérisation.

12. Un procédé selon la revendication 11, caractérisé en ce que la teneur en eau de ladite résine de polyacrylate réticulée n'est pas supérieure à environ 10 % en poids à l'état récupéré de la solution monomère mixte après polymérisation, sans une étape additionnelle de séchage.

13. Un procédé selon l'une quelconque des revendications précédentes, caractérisé par le dépôt dudit mélange monomère incluant ledit amorceur sur une surface de support sous la forme d'une feuille pour la polymérisation et la réticulation.

14. Une méthode pour absorber de l'eau par préparation d'une résine solide absorbant l'eau conformément à l'une quelconque des revendications précédentes et ensuite mise en contact de ladite résine avec l'eau pour absorber l'eau dans ladite résine.

## Patentansprüche

1. Verfahren zur Herstellung eines festen, wasserabsorbierenden Harzes, **dadurch gekennzeichnet,** daß es das Mischen einer Monomerlösung aus (A) Acrylsäure, 70 bis 100 Mol-% neutralisiert; und (B) einem wassermischbaren bis wasserlöslichen Polyvinylmonomer in einer kombinierten Konzentration aus wenigstens 30 Gew.-% mit Wasser zur Bildung einer gemischten Monomerlösung und das Einleiten einer Polymerisation aus den Monomeren (A) und (B) auf solche Weise, daß während der Polymerisation die exotherme Reaktionswärme im wesentlichen die einzige Wärmeenergie ist, die zur Durchführung der Polymerisation verwendet wird, das Vernetzen und Abziehen von ausreichend

Wasser, um ein festes, vernetztes Harz mit einem Wassergehalt von 15 Gew.-% oder weniger zu erhalten, umfaßt.

2. Verfahren nach Anspruch 1, **dadurch gekennzeichnet,** daß die Komponente (A) Kaliumacrylat ist.

3. Verfahren nach Anspruch 1 oder 2, **dadurch gekennzeichnet,** daß die kombinierte Konzentration der Monomeren (A) und (B) wenigstens 30 Gew.-% und weniger als 70 Gew.-% beträgt.

4. Verfahren nach einem der vorhergehenden Ansprüche, **dadurch gekennzeichnet,** daß die gemischte Monomerlösung eine Temperatur von 50 bis 85°C vor der Polymerisation besitzt.

5. Verfahren nach einem der vorhergehenden Ansprüche, **dadurch gekennzeichnet,** daß das Monomer (B) N,N-Methylenbisacrylamid oder N,N-Methylenbismethacrylamid umfaßt.

6. Verfahren nach einem der vorhergehenden Ansprüche, **dadurch gekennzeichnet,** daß die gemischte Monomerlösung 1 bis 10 Gew.-% eines organischen Lösungsmittels, bezogen auf das Gewicht der Monomere (A) und (B), enthält.

7. Verfahren nach einem der vorhergehenden Ansprüche, **dadurch gekennzeichnet,** daß die gemischte Monomerlösung weiterhin ein organisches Lösungsmittel mit einem Siedepunkt von 40 bis 150°C enthält.

8. Verfahren nach einem der vorhergehenden Ansprüche, **dadurch gekennzeichnet,** daß der Polymerisationsinitiator in einer Menge von wenigstens 0,5 Gew.-% der Monomere (A) und (B) zugegeben wird.

9. Verfahren nach einem der vorhergehenden Ansprüche, **dadurch gekennzeichnet,** daß der Wassergehalt des vernetzten Harzes nicht größer als etwa 10 Gew.-%, gewonnen aus der polymerisierten Mischung, ohne zusätzliche Trocknungsstufe ist.

10. Verfahren nach einem der vorhergehenden Ansprüche, **gekennzeichnet** durch die Stufe des Pulverisierens des vernetzten Harzes zur Bildung eines Pulvers.

11. Verfahren nach einem der vorhergehenden Ansprüche, **dadurch gekennzeichnet,** daß die gemischte Monomerlösung (A) Acrylsäure, 70 bis 100 Mol-% neutralisiert, (B) Acrylamid in einem Mol-Verhältnis von (A):(B) in einem Bereich von 70:30 bis 100:0, (C) ein wasserlösliches oder wassermischbares Polyvinylmonomervernetzungsmittel in einer Menge von 0,001 bis 0,3 Gew.-% von (A) plus (B), und Wasser umfaßt, worin die Monomerkonzentration unterhalb 70 Gew.-% der Monomerlösung vor Beginn der Polymerisation liegt.

12. Verfahren nach Anspruch 11, **dadurch gekennzeichnet,** daß der Wassergehalt des vernetzten Polyacrylatharzes nicht größer als etwa 10 Gew.-% ist, gewonnen aus der gemischten Monomerlösung nach der Polymerisation, ohne zusätzliche Trocknungsstufe.

13. Verfahren nach einem der vorhergehenden Ansprüche, **gekennzeichnet durch** Abscheiden der Monomermischung, einschließlich des Initiators, auf einer Trägeroberfläche in Plattenform zur Polymerisation und Vernetzung.

14. Verfahren zum Absorbieren von Wasser durch Herstellen eines festen, wasserabsorbierenden Harzes gemäß einem der vorhergehenden Ansprüche und anschließendes In-Berührung-Bringen des Harzes mit Wasser zur Absorption von Wasser in dem Harz.